# EUROPÄISCHE PATENTSCHRIFT

(11) **EP 0 549 618 B1**
(45) Veröffentlichungstag und Bekanntmachung des Hinweises auf die Patenterteilung: **22.11.1995**
(21) Anmeldenummer: 91915450.0
(22) Anmeldetag: 09.09.1991
(51) Int. Cl.: C07C 43/184, C07C 41/20

(54) **VERFAHREN ZUR HERSTELLUNG VON TRANS-3,3,5-TRIMETHYLCYCLOHEXYLETHYLETHER**
METHOD FOR THE PREPARATION OF TRANS-3,3,5-TRIMETHYLCYCLOHEXYL ETHYL ETHER
PROCEDE DE PRODUCTION DE TRANS-3,3,5-TRIMETHYLCYCLOHEXYLETHYLETHER

(30) Priorität: 17.09.1990 DE 4029425
(43) Veröffentlichungstag der Anmeldung: 07.07.1993
(73) Patentinhaber: Henkel Kommanditgesellschaft auf Aktien, 40191 Düsseldorf (DE)
(72) Erfinder: BEHR, Arno, D-4000 Düsseldorf 13 (DE); LOHR, Christoph, D-4600 Dortmund (DE); HANDWERK, Hans-Peter, D-4000 Düsseldorf 13 (DE); GERKE, Thomas, D-4040 Neuss 1 (DE)
(86) Internationale Anmeldenummer: EP9101709
(87) Internationale Veröffentlichungsnummer: WO9205136

(56) Entgegenhaltungen:
- DE-A-12 658 567
- DE-C-22 661 006
- Bulletin of the chemical society of Japan, Band. 51. Nr.11,1978 Shigeo Nishimura et al.: "Hydrogenation and Hydrogenolysis. XVI.The Reactions of Two Isomeric Enol Ethers of 3-Methylcyclohexanone over Platinum Group Metals", siehe Seite 3330- Seite 3334

## Beschreibung

Die Erfindung betrifft ein verbessertes Verfahren zur Herstellung des Riechstoffs trans-3,3,5-Trimethylcyclohexyl-ethylether durch Hydrierung des Enolethers 3,3,5-Trimethylcyclohexenyl-ethylether in guter Ausbeute und hoher Selektivität unter besonders milden Reaktionsbedingungen.

Die Hydrierung von Enolethern ist grundsätzlich literaturbekannt. Beispielsweise haben A.Yanagawa, Y.Suzuki, I.Anazawa, Y.Takagi und S.Yada Methyl-4-tert.-butyl-1-cyclohexenylether hydriert. Sie erhielten überwiegend das cis-Isomer, wenn der Katalysator metallisches Rhodium enthielt, und überwiegend das trans-Isomer, wenn der Katalysator einen Rhodiumkomplex enthielt (J.Mol.Catal. 1985 (29) 41).

S.Nishimura, M.Katagiri, T.Watanabe und M.Uramoto haben gezeigt, daß die Pd-katalysierte Hydrierung der Enolether von 2- und 4-Methylcyclohexanon mit hoher Stereoselektivität zu den gesättigten cis-Ethern führt (Bull.Chem.Soc.Jpn 1971 (44) 166).

In einer weiteren Arbeit haben S.Nishimura, K.Kagawa und N.Sato (Bull.Chem.Soc.Jpn 1978 (51) 3330) nachgewiesen, daß die entsprechenden isomeren Enolether von 3-Methylcyclohexanon, 1-Ethoxy-3-Methyl- und 1-Ethoxy-5-Methyl-cyclohexen, bei der Pd-katalysierten Hydrierung in Ethanol in der Anfangsphase der Reaktion überwiegend zu den trans-Ethern führen. Die cis-Anteile betrugen dabei jedoch immerhin noch 23% bzw. 15%. Die Reaktion hat darüber hinaus den Nachteil, daß sie in einem Lösungsmittel durchgeführt werden muß. Dies kompliziert einerseits die Aufarbeitung des Produktes in unnötiger Weise, andererseits kann es Anlaß zur Bildung von Nebenprodukten geben.

Nach der Lehre der deutschen Patentschrift DE 26 61 006 C2 ist 3,3,5-Trimethylcyclohexyl-ethylether **(1)** durch Hydrierung von 3,3,5-Trimethylcyclohexenyl-ethylether ("Enolether") mittels Nickelkatalysator in einer Ausbeute von 75 % der Theorie zugänglich. Dabei wird die gewünschte trans-Form des Ethers mit 95 %-iger Selektivität gebildet. Zur Durchführung dieser Reaktion bedarf es jedoch energischer Bedingungen hinsichtlich Druck und Temperatur. Gemäß den Ansprüchen der o.g. Patentschrift wird die Reaktion bei 150 bis 200 °C und Wasserstoffdrucken von 10 bis 200 bar durchgeführt. 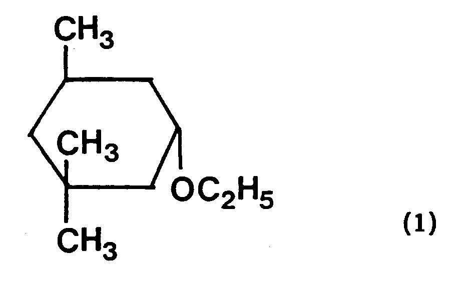
Aus Gründen der Senkung des Energiebedarfs, des Gefahrenpotentials bei der Handhabung von Wasserstoffgas unter energischen Bedingungen sowie der Erhöhung der Lebensdauer der Reaktoren wurde zur Herstellung von **(1)** nach einem Verfahren gesucht, das bei mindestens gleicher Selektivität unter wesentlich milderen Bedingungen durchführbar ist. Darüber hinaus war aus Gründen der Ökonomie eine Verbesserung der Ausbeute bei der Hydrierung des Enolethers bei möglichst geringen Katalysatorkonzentrationen anzustreben. Schließlich sollte das Verfahren unter Bedingungen durchführbar sein, die die Anwesenheit eines Lösungsmittels entbehrlich machen.

Diese Aufgaben wurden erfindungsgemäß gelöst durch ein Verfahren zur Herstellung von trans-3,3,5-Trimethylcyclohexylethylether mit nur geringen Beimengungen an cis-Ether, dadurch gekennzeichnet, daß man 3,3,5-Trimethylcyclohexenyl-ethylether in Gegenwart von Palladiumkatalysatoren bei 20 bis 100 °C und bei 1 bis 10 bar Wasserstoffdruck ohne Einsatz eines Lösungsmittels hydriert und das Hydrierungsprodukt in der üblichen Weise aufarbeitet.

Eine bevorzugte Ausführungsform der Erfindung ist dadurch gekennzeichnet, daß als Katalysator Palladium auf Kohle verwendet wird.

Es ist dabei besonders bevorzugt, die Hydrierung bei 20 bis 50 °C durchzuführen.

Eine weitere bevorzugte Ausführungsform der vorliegenden Erfindung besteht darin, daß die Hydrierung bei Wasserstoffdrucken von 1 bis 6 bar durchgeführt wird.

Bei der Hydrierung des 3,3,5-Trimethylcyclohexenyl-ethylethers nach dem erfindungsgemäßen Verfahren kann bei sehr niedrigen Katalysatorkonzentrationen gearbeitet werden. Eine weitere bevorzugte Ausführungsform der Erfindung ist daher dadurch gekennzeichnet, daß das molare Verhältnis von Palladium und 3,3,5-Trimethylcyclohexenyl-ethylether 1 : 1000 bis 1 : 15000, bevorzugt 1 : 1000 bis 1 : 5000, beträgt.

Das erfindungsgemäße Verfahren hat darüber hinaus den Vorteil, daß der Katalysator sich leicht abfiltrieren läßt und ohne nennenswerten Aktivitätsverlust mehrfach wiederverwendet werden kann, ohne daß dabei Einbußen in bezug auf Ausbeute und Stereoselektivität hingenommen werden müssen.

Die Hydrierung unter den Bedingungen des erfindungsgemäßen Verfahrens stellt gegenüber dem Stand der Technik Verbesserungen in folgenden Punkten dar:
- sie verläuft quantitativ
- die gewünschte trans-Form des 3,3,5,-Trimethylcyclohexyl-ethylethers wird mit Selektivitäten von 97 bis 98 % gebildet
- sie läuft bei wesentlich tieferen Temperaturen ab
- sie läuft bei wesentlich geringeren Drucken ab
- sie erfordert kein Lösungsmittel
- sie verläuft bereits bei sehr niedrigen Katalysator-Konzentrationen
- der Katalysator läßt sich ohne Einbußen in bezug auf Ausbeute und Stereoselektivität mehrfach wiederverwenden
Die folgenden Beispiele sollen den Gegenstand der Erfindung näher erläutern, ohne ihn hierauf zu beschränken.

### Beispiele

3,3,5,-Trimethylcyclohexenyl-ethylether ("Enolether") wurde nach der Vorschrift der deutschen Patentschrift DE 26 61 006 C2 aus 3,3,5-Trimethylcyclohexanon und Orthoameisensäuretriethylester in Gegenwart von wenig p-Toluolsulfonsäure hergestellt.

### Vergleichsbeispiel 1 (gemäß DE 26 61 006 C2)

Reaktion: 252 g (1,5 mol) Enolether wurden mit 18,4 g Katalysator (Girdler Nickel 49A ^{R} ) versetzt und im Autoklaven unter 50 bar Wasserstoffdruck langsam auf 200 °C erhitzt. Anschließend ließ man eine Stunde bei einem Wasserstoffdruck von 180 bar nachreagieren.

Aufarbeitung: Das Gemisch wurde abgekühlt, das Produkt vom Katalysator abdekantiert und anschließend im Vakuum fraktioniert.

Ausbeute: 184 g 3,3,5-Trimethylcyclohexyl-ethylether (74 % d.Th.)

Reinheit: 95 % trans-3,3,5-Trimethylcyclohexyl-ethylether
5 % cis-3,3,5-Trimethylcyclohexyl-ethylether

### Beispiel 1

Reaktion: 252 g (1,5 mol) Enolether wurden in einem mit Turbinenrührer ausgestatteten Laborautoklaven vorgelegt und mit 2,4 g (1,1 mmol Pd) Katalysator (5 % Palladium auf Aktivkohle) versetzt. Anschließend wurde der Autoklav mit 6 bar H₂ beaufschlagt und die Reaktion gestartet. Im Zuge der exothermen Reaktion erhöhte sich die Temperatur der Reaktionsmischung von 25 auf 50 °C. Nach 60 Minuten betrug die Ausbeute an trans-3,5,5-Trimethylcyclohexyl-ethylether 90%. Es wurde weitere 40 Minuten bei konstantem Wasserstoffdruck auf 100 °C nachgerührt.

Aufarbeitung: wie in Vergleichsbeispiel 1

Ausbeute: 250 g g 3,3,5-Trimethylcyclohexyl-ethylether (quantitativ)

Reinheit: 98 % trans-3,3,5-Trimethylcyclohexyl-ethylether
2 % cis-3,3,5-Trimethylcyclohexyl-ethylether

### Beispiel 2

Reaktion: Analog zu Beispiel 1, jedoch bei konstantem Wasserstoffdruck von 2 (statt 6) bar und einer Nachrührzeit von 70 (statt 40) Minuten.

Aufarbeitung: wie in Beispiel 1.

Ausbeute: 250 g 3,3,5-Trimethylcyclohexyl-ethylether (quantitativ)

Reinheit: 97 % trans-3,3,5-Trimethylcyclohexyl-ethylether
3 % cis-3,3,5-Trimethylcyclohexyl-ethylether

### Beispiel 3

Reaktion: Analog zu Beispiel 1, jedoch bei konstantem Wasserstoffdruck von 4 (statt 6) bar und einer Nachrührzeit von 50 (statt 40) Minuten.

Aufarbeitung: wie in Beispiel 1.

Ausbeute: 250 g 3,3,5-Trimethylcyclohexyl-ethylether (quantitativ)

Reinheit: 97 % trans-3,3,5-Trimethylcyclohexyl-ethylether
3 % cis-3,3,5-Trimethylcyclohexyl-ethylether

### Beispiel 4

Reaktion: Analog zu Beispiel 1, jedoch mit 0,218 g (0,1 mmol Pd) Katalysator.

Aufarbeitung: wie in Beispiel 1.

Ausbeute: 250 g 3,3,5-Trimethylcyclohexyl-ethylether (quantitativ)

Reinheit: 97 % trans-3,3,5-Trimethylcyclohexyl-ethylether
3 % cis-3,3,5-Trimethylcyclohexyl-ethylether

### Beispiel 5

Reaktion: Analog zu Beispiel 1, jedoch unter Verwendung eines Katalysators, der unter gleichen Reaktionsbedingungen bereits 14-mal eingesetzt worden war.

Aufarbeitung: wie in Beispiel 1.

Ausbeute: 250 g 3,3,5-Trimethylcyclohexyl-ethylether (quantitativ)

Reinheit: 97 % trans-3,3,5-Trimethylcyclohexyl-ethylether
3 % cis-3,3,5-Trimethylcyclohexyl-ethylether

## Patentansprüche

1. Verfahren zur Herstellung von trans-3,3,5-Trimethylcyclohexyl-ethylether mit nur geringen Beimengungen an cis-Ether, dadurch gekennzeichnet, daß man 3,3,5-Trimethylcyclohexenyl-ethylether in Gegenwart von Palladiumkatalysatoren bei 20 bis 100 °C und bei 1 bis 10 bar Wasserstoffdruck ohne Einsatz eines Lösungsmittels hydriert und das Hydrierungsprodukt in der üblichen Weise aufarbeitet.

2. Verfahren gemäß Anspruch 1, dadurch gekennzeichnet, daß als Katalysator Palladium auf Kohle verwendet wird.

3. Verfahren gemäß Anspruch 1 oder 2, dadurch gekennzeichnet, daß die Hydrierung bei 20 bis 50 °C durchgeführt wird.

4. Verfahren gemäß mindestens einem der Ansprüche 1 bis 3, dadurch gekennzeichnet, daß die Hydrierung bei Wasserstoffdrucken von 1 bis 6 bar durchgeführt wird.

5. Verfahren gemäß mindestens einem der Ansprüche 1 bis 4, dadurch gekennzeichnet, daß das molare Verhältnis von Palladium und 3,3,5-Trimethylcyclohexenyl-ethylether 1 : 1000 bis 1 : 15000, bevorzugt 1 : 1000 bis 1 : 5000, beträgt.

## Claims

1. A process for the production of trans- 3,3,5-trimethylcyclohexyl ethyl ether with only small contents of cis-ether, characterized in that 3,3,5-trimethylcyclohexenyl ethyl ether is hydrogenated in the presence of palladium catalysts at 20 to 100°C/1 to 10 bar hydrogen pressure without using a solvent and the hydrogenation product is worked up in the usual way.

2. A process as claimed in claim 1, characterized in that palladium on carbon is used as the catalyst.

3. A process as claimed in claim 1 or 2, characterized in that the hydrogenation is carried out at 20 to 50°C.

4. A process as claimed in at least one of claims 1 to 3, characterized in that the hydrogenation is carried out under hydrogen pressures of 1 to 6 bar.

5. A process as claimed in at least one of claims 1 to 4, characterized in that the molar ratio of palladium to 3,3,5-trimethylcyclohexenyl ethyl ether is 1:1000 to 1:15000 and preferably 1:1000 to 1:5000.

## Revendications

1. Procédé de production de trans-3,3,5-triméthylcyclohéxyl-éthyléther avec seulement de faibles quantités associées d'éther-cis, caractérisé en ce qu'on hydrogène le 3,3,5-triméthylcyclohéxyl-éthyléther en présence de catalyseurs au palladium entre 20 et 100°C et sous une pression de 1 à 10 bars d'hydrogène sans utiliser de solvant, et on traite le produit d'hydrogénation de manière habituelle.

2. Procédé selon la revendication 1, caractérisé en ce qu'on utilise comme catalyseur du palladium sur charbon actif.

3. Procédé selon la revendication 1 ou 2, caractérisé en ce qu'on réalise l'hydrogénation entre 20 et 50°C.

4. Procédé selon au moins l'une des revendications 1 à 3, caractérisé en ce qu'on réalise l'hydrogénation sous des pressions d'hydrogène de 1 à 6 bars.

5. Procédé selon au moins l'une des revendications 1 à 4, caractérisé en ce que la proportion molaire du palladium au 3,3,5-triméthylcyclohéxényl-éthyléther est 1:1000 à 1:1500, de préférence 1:1000 à 1:5000.
